**Europäisches Patentamt**

**European Patent Office**

**Office européen des brevets**

(19)

(11) Veröffentlichungsnummer: **0 124 834**
**A2**

(12) # EUROPÄISCHE PATENTANMELDUNG

(21) Anmeldenummer: **84104748.3**

(22) Anmeldetag: **27.04.84**

(51) Int. Cl.³: **A 61 F 13/00**
**A 61 F 13/18, A 61 F 13/20**

(30) Priorität: **05.05.83 DE 3316431**

(43) Veröffentlichungstag der Anmeldung:
**14.11.84** Patentblatt **84/46**

(84) Benannte Vertragsstaaten:
**AT BE CH DE FR GB IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Sustmann, Scarlet, Dr.**
**Am Nachtigallenwäldchen 34**
**D-4060 Viersen 12(DE)**

(54) **Hygienevlies und Verfahren zum Herstellen des Vlieses.**

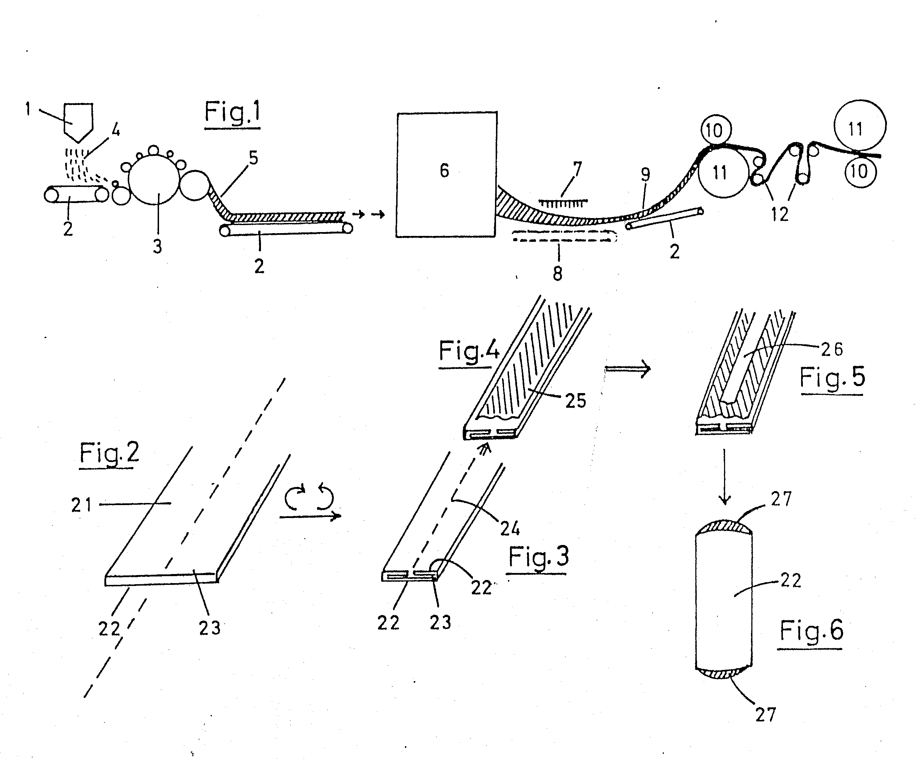

(57) Um ein aus einem Watteband bestehendes Hygienevlies ohne das Erfordernis eines separaten Vliesstoffs flusendicht zu umhüllen, wird das Ausgangsmaterial mit einem zum thermoplastischen Verfestigen der Oberfläche ausreichenden Kunststoffaseranteil versetzt und eine Fläche des vorbereiteten Wattebandes in einem Kalander mit mindestens einer eine beheizte Prägestruktur aufweisenden Arbeitswalze (10) vliesartig verfestigt (Figur 1).

Fig. 1

EP 0 124 834 A2

Patentanmeldung
Henkelstr. 67
4000 Düsseldorf, den 3. 5. 1983

HENKEL KGaA
ZR-FE/Patente
Bor/C

0124834

P a t e n t a n m e l d u n g
D 6837

## "Hygienevlies und Verfahren zum Herstellen des Vlieses"

Die Erfindung betrifft ein Hygienevlies bestehend aus einem mit einem flusendichten Vliesstoff bedeckten Watteband. Sie betrifft ferner ein Verfahren zum Herstellen eines solchen Hygienevlieses.

Zur Aufnahme von Körperflüssigkeit, z.B. in Wundauflagen, Slipeinlagen sowie in zur Aufnahme von Menstrualflüssigkeit dienenden Vorlagen, insbesondere sogenannten Tampons, werden überwiegend Wattebandvliese verwendet. Bei der Herstellung wird das Watteband entweder durch Kalandrieren oder durch Vernadeln verfestigt und anschließend zu einem zylindrischen Körper verpreßt. Da das Ausgangsmaterial aus einzelnen Fasern von durchschnittlich 20 bis 40 mm Länge besteht, ist ein Zurückbleiben von Fasern bzw. Watteteilen in der Wunde, Vagina usw. nach Entfernen des Tampons nicht auszuschließen. Zurückbleibende Teile der Watte können zu einer Entzündung führen.

Es wurde deshalb versucht, ein Abflusen durch gesonderten Einschlag des Wattebandes in Vliesmaterial zu unterbinden. Bei einem solchen Verfahren wird ein Watteband mit einem Vliesstoff, Synonyme oder dgl., überlappend eingehüllt. Bei der Tampon-Herstellung werden daraufhin Wattebandabschnitte abgetrennt und nach Aufbringen der Rückholkordel entweder linear oder nach 90° Umlenkung in eine Preßkammer eingeführt. Die auf diese Weise erhaltenen Tampons sind entweder an den Längsseiten voll umhüllt und an den Kopfenden offen oder sie sind an den Kopfenden umhüllt

...

und besitzen eine in Längsrichtung verlaufende offene
Schnittfläche.

In den DE-OS 32 36 540 und 32 36 541 wurden bereits
Verfahren zum Vollumhüllen eines Tampons vorgeschlagen.
Im ersteren Fall wird eine Vollumhüllung des Tampons dadurch erreicht, daß die nach dem Umhüllen des Wattebandes
mit einem flusenfreien Material erzeugten freien Schnittkanten auf die Fläche des umhüllten Wattebandes umgelegt
und dort verpreßt werden. Bei dem Herstellungsverfahren
wird dazu ein entsprechender Arbeitsgang eingeschaltet,
bei dem der abgetrennte und umhüllte Wattebandabschnitt
auf einem Führungsband weiterläuft und dort mit Hilfe
von Leitblechen an den freien Schnittkanten umgelegt und
verpreßt wird. In dem Verfahren nach der zweiten Offenlegungsschrift werden die nach dem Einlegen des Wattebandes in das Hüllmaterial sowie Abtrennen in Einzelstücke offenbleibenden Kanten versiegelt.

Allen bekannten Verfahren ist gemeinsam, daß ein separater Vliesstoff zur Umhüllung des Wattebandes benötigt
wird. Dieses Erfordernis ist sowohl hinsichtlich des
Aufwandes an Material als auch der technischen Installationen ungünstig.

Der Erfindung liegt die Aufgabe zugrunde, ein nicht flusendes Hygienevlies zu schaffen, das bei ausgezeichneter Saugfähigkeit ohne separate Vliesumhüllung auskommt
und kostengünstig herzustellen ist. Die erfindungsgemäße
Lösung ist bei dem eingangs genannten Hygienevlies gekennzeichnet durch eine heißgeprägte Oberflächenverfestigung eines thermoplastischen Faseranteils des Wattebandes als Vliesstoffdeckschicht.

...

Gemäß weiterer Erfindung ist ein Verfahren zum Herstellen eines Hygienevlieses bestehend aus einem mit einem flusendichten Vliesstoff bedeckten Watteband dadurch gekennzeichnet, daß ein Watteband mit einem zum thermoplastischen Verfestigen der Oberfläche ausreichenden Kunststoffaseranteil vorbereitet wird und daß wenigstens eine Fläche des vorbereiteten Wattebandes in einem Kalander mit mindestens einer eine beheizte Prägestruktur aufweisenden Arbeitswalze vliesartig verfestigt wird. Verbesserungen und weitere Ausgestaltungen der Erfindung werden in den Unteransprüchen angegeben.

Die Tatsache, daß das vorbereitete Watteband vor dem Einlauf in die die entsprechenden Abschnitte bildenden Tamponmaschine oder dergleichen einen beheizten Kalander durchläuft, hat auf der so behandelten Oberfläche des Wattebandes eine "in situ"-Vliesbildung, bei der das Vlies Bestandteil des Wattebandes bleibt und fest daran gebunden ist, zur Folge. Je nach Anwendungszweck können auf diese Weise auch beide Oberflächen des Wattebandes in ein flusendichtes Deckvlies verwandelt werden. Schließlich können nach üblichen Verfahren auch die Schnittkanten und Seitenkanten des Bandes mit einem Thermoschneidverfahren flusendicht abgeschlossen werden.

Der zum Bilden einer flusendichten Vliesoberfläche auf der Ober- und/oder Unterseite des Wattebandes vorgesehene Kalander soll vorzugsweise zwei oder vier Walzen besitzen, wobei zu jedem Walzenpaar eine beheizte Walze mit Prägestruktur, z.B. mit rautenförmiger oder sechseckiger Struktur, und eine Gegenwalze gehören. Die Temperatur der beheizten Prägewalzen bzw. Arbeitswalzen wird den eingesetzten thermoplastischen Fasern entsprechend vorgegeben. Bei

...

Patentanmeldung

HENKEL KGaA
ZR-FE/Patente

D 6837                           4

Verwendung eines 20 %igen Anteils von Polypropylenfasern
sind Prägetemperaturen zwischen 150 und 160°C geeignet.
Der Preßdruck des Kalanders wird zweckmäßig bei größerer
Durchlaufgeschwindigkeit erhöht und bei kleinerer Durchlaufgeschwindigkeit vermindert. Er kann in der Größenordnung von 0,5 bis 2 bar liegen. Die Struktur der Präge- bzw. Arbeitswalzen wird ebenfalls dem zu bearbeitenden Material entsprechend ausgewählt. Die erhabenen
Strukturen können beispielsweise einen Durchmesser von
0,5 bis 3 mm besitzen.

Zum Herstellen eines Wattebandes für die Tamponfertigung
wird beispielsweise eine Mischung von 80 % üblicher
Tamponwatte, z.B. 100 % Zellwolle, bevorzugt mit einem
dtex von 2,8 bis 3,6 und einer Faserlänge von 30 bis
40 mm sowie ca. 20 % einer thermoplastischen Faser verwendet. Das Mischen der Fasern kann in einem Wägekastenspeiser ausgeführt werden. Die Weiterverarbeitung erfolgt dann über die Krempeltechnik mit anschließendem
Kalandrieren zu einem Watteband der gewünschten Breite,
z.B. 50 bis 90 mm.

Statt nach der Krempeltechnik läßt sich das Watteband
auch nach der Wirrlagentechnik, insbesondere mit anschließendem Vernadeln, erzeugen. Bei der Tamponherstellung ist es erfindungsgemäß an dieser Stelle wesentlich,
daß das Watteband vor dem Einlauf in die Tamponmaschine,
welche das Watteband zu entsprechenden Abschnitten zerteilt und anschließend zum fertigen Tamponkörper verpreßt, den beheizten, wenigstens eine Prägewalze aufweisenden Kalander durchläuft.

Die erfindungsgemäße Walzenprägung mit geprägten Linien
und Punkten hat auch maßgeblichen Einfluß auf die Weich-

...

D 6837                           5

heit der Oberfläche. Demgegenüber würde eine vollflächige Verfestigung zu relativ steifen und nicht gut verpreßbaren Wattebandabschnitten führen. Auch die Saugfähigkeit wird durch eine "punktförmige" Verfestigung verbessert.

Es können auch die Kanten der Wattebandabschnitte, insbesondere nach dem Thermoschneideverfahren, verfestigt werden. Aber auch nicht auf diese Weise versiegelte Schnittkanten lassen sich selbstverständlich durch spezielle Faltverfahren, wie sie in den eingangs genannten Offenlegungsschriften beschrieben werden, in das Innere des Tampons oder dergleichen verlegen.

Nach dem erfindungsgemäßen Verfahren können außer Tampons auch Slipeinlagen, Wundauflagen und dergleichen hergestellt werden. Statt ein Watteband mit separatem Vliesstoff zu umhüllen, wird durch das erfindungsgemäße Heißprägesystem ein einseitig oder beidseitig oberflächenverfestigtes, quasi mit einem Vliesstoff verwachsenes Watteband hergestellt. Bei der Produktion von Slipeinlagen werden beispielsweise erfindungsgemäße Vliesstoffbänder von 110 bis 200 g/m$^2$ erzeugt, U-förmig eingeschlagen und einseitig mit einem flüssigkeitsundurchlässigen Wäscheschutz, z.B. mit einer PP- oder PE-Folie, abgedeckt. Die Enden werden durch übliches Präge/Stanzverfahren gebildet. Es ist auch möglich, aus einem Band bzw. Flächengebilde Einlagen nach den Siegel-Präge/Stanzverfahren zu erzeugen. Wenn mit einer Rotationswalze mit mehreren vorgegebenen Stanzmustern gearbeitet wird, lassen sich auch entsprechend viele Einlagen nebeneinander rationell herstellen.

Patentanmeldung

D 6837                     6

Anhand von Ausführungsbeispielen und der Zeichnung werden weitere Einzelheiten der Erfindung erläutert. Dabei zeigt:

Fig. 1    den Herstellungsgang eines Wattebandes
          mit flusendichter Oberflächenschicht;
          und

Fig. 2
bis 6     Stufen beim Herstellen von Slipeinlagen.

Beispiel I: Tampon-Herstellung

1. Wattebandherstellung

Es wurde eine Fasermischung aus 80 % Zellwolle mit 3,6 dtex, 30 mm Faserlänge und 20 % Polypropylenfaseranteil (3,0 den, 1 7/8", matt) nach einem üblichen Auflöseverfahren, insbesondere nach der Krempeltechnik, hergestellt und hieraus ein Watteband mit einem Gewicht von 728 $g/m^3$ gebildet und anschließend durch Vernadelung verfestigt.

Gemäß Fig. 1 wird eine Zellwolle/Polypropylen-Mischung 4 aus einem Wägekastenspeiser 1 über ein Transportband 2 auf einen Krempel 3 mit Arbeitern und Wendern transportiert. Der Faserflor 5 gelangt über weitere Transportbänder 2 durch ein Aufbereitungsgerät 6 zur Wirrlagenbildung und schließlich auf ein Siebband 8, wo er mit einem Nadelgerät 7 behandelt wird. Das vernadelte (alternativ auch kalandrierte) Watteband 9 läuft nun über ein Transportband 2 in einen beheizbaren Kalander, der aus einer beheizten Stahlgravurwalze 10 und einer Baumwoll- oder Kunststoffwalze 11 besteht.

D 6837                              7

Bei der Erprobung erwies sich ein aus einer beheizten
Stahl-Gravurwalze mit feiner Rasterprägung mit gegenüberliegender Baumwollwalze bestehender Kalander als besonders günstig. Die Temperatur betrug bei den oben genannten Materialien 158°C und der Druck 0,5 bar bei einer
Laufgeschwindigkeit von 3 m pro Minute. Es wurde eine
weiche durchgehende Oberflächenverfestigung auf dem Watteband 9 erzielt.

Das Watteband konnte beidseitig oberflächlich verfestigt
werden, wenn im Anschluß an den ersten Kalander, gegebenenfalls nach Führung über Umlenkrollen 12 ein weiterer
Kalander mit nunmehr auf die bereits verfestigte Fläche
des Wattebandes 9 wirkender Gegenwalze und auf die unverfestigte Wattebandfläche wirkender Stahlgravurwalze 12
nachgeschaltet wird. Im Ausführungsbeispiel hatte der
oberflächenverfestigte Wattebandanteil ein Gewicht von ca. 15
bis 20 g/m$^2$. Ein Abflusen trat nicht mehr auf.

2.  Tamponherstellung

Aus dem nach vorbeschriebenem Ausführungsbeispiel oberflächenverfestigten Watteband wurden Abschnitte von
40x70 mm gefertigt und dabei zum Teil mit Hilfe einer
Schneid/Siegelvorrichtung auch die Schnittkanten verfestigt. In Längsrichtung der Wattebandabschnitte wurde
je eine Rückholkordel aufgenäht. Sodann wurden die Abschnitte axial und radial nach üblichen Verfahren zu zylindrischen Körpern verpreßt und in Einführhülsen eingeschoben. Die Preßtemperatur betrug 100 bis 120°C.

...

3. Anwendungstechnische Prüfung

Die aus dem oberflächenverfestigten Watteband hergestellten Tampons wurden mit solchen aus gleicher Fasermischung ohne Oberflächenverfestigung und mit herkömmlichen Tampons aus 100 % Zellwolle (3,6 dtex, 30 mm)
verglichen.

Das Herstellungsverfahren war bis auf die Oberflächenverfestigung bei allen drei Tamponvariationen gleich.
Geprüft wurde nach Entnahme der Tampons aus der Einführhülse die Geometrie und die Saugfähigkeit. Diese Daten
werden in der nachfolgenden Tabelle zusammengefaßt. In
der Tabelle bezieht sich die erste Zeile auf einen aus
einem erfindungsgemäßen oberflächenverfestigten Watteband hergestellten Tampon. Die zweite Zeile bezieht sich
auf einen Tampon mit gleichen Ausgangsgrößen wie die erste aber ohne erfindungsgemäße Oberflächenverfestigung.
Die dritte Zeile bezieht sich auf einen Tampon aus 100 %
Zellwolle, 3,6 dtex, 30 mm.

Die Bestimmung der Saugkapazität erfolgte mit Hilfe einer
künstlichen Vagina. Dieses Testgerät wird als Syngina
bezeichnet. Als Testflüssigkeit dient Wasser bei einem
Anfangsdruck von 30 mbar. Es wurde nicht isobar gearbeitet, vielmehr stieg der Druck mit zunehmender Quellung bei Flüssigkeitsbeaufschlagung. In der Tabelle bedeuten V das Tamponvolumen und G die Tampondichte. Bei
gleichem Wattegewicht hat ein erfindungsgemäß hergestellter Tampon ein geringfügig geringeres Volumen als
ein solcher ohne Oberflächenverfestigung und demgemäß
ist die Dichte etwas größer, aber die Saugfähigkeit und
der Druckzuwachs sind bei dem erfindungsgemäß hergestellten Tampon wesentlich besser als in allen anderen Fällen.

...

| | Wattegew. (g) | Tamponlänge (mm) | Durchmesser (mm) | $V$ $(cm^3)$ | $G$ $(g/cm^3)$ | Saugkapazität, Syngina 30 mbar Druck steigend, Testflüssigkeit: $H_2O$ | | |
|---|---|---|---|---|---|---|---|---|
| | | | | | | ml/Tampon | ml/g | Druckzuwachs |
| 1. | 2,75 | 48,2 | 15,4 | 8,96 | 0,307 | 10,75 | 3,91 | 5,0 |
| 2. | 2,75 | 52,1 | 15,1 | 9,36 | 0,294 | 10,2 | 3,70 | 2,2 |
| 3. | 2,75 | 46,7 | 12,7 | 5,95 | 0,462 | 9,1 | 3,31 | 6,8 |

HENKEL KGaA
ZR-FE/Patente
0124834

4. Ergebnisse

Die Tampons mit 20 % Polypropylen entspannen sich nach Entnahme aus der Hülse und nehmen ein größeres Volumen V als Tampons aus reiner Zellwolle an. Dies ist als Vorteil zu werten, da die nach Entspannung flexiblen und relativ weichen Tampons sich gut an die vaginalen Wände anschmiegen und ein Vorbeidringen von Flüssigkeit wirkungsvoll verhindern. Demgegenüber behalten die Tampons aus 100 % Zellwolle zunächst ihre harte stäbchenförmige Gestalt und beginnen erst nach Kontakt mit Flüssigkeit zu quellen.

Die Vliesbildung beeinträchtigt nicht die Saugkapazität. Im Vergleich zu Tampons aus 100 % Zellwolle ist die Saugkapazität der Tampons mit Polypropylen um ca. 18 % erhöht. Dies ist u.a. auf die größere Rückstellkraft dieser Fasermischung zurückzuführen, so daß Flüssigkeit rascher aufgenommen werden kann. Selbstverständlich nimmt in diesem Bereich auch die Saugfähigkeit umgekehrt proportional zur Dichte zu. Der Druckzuwachs bei Flüssigkeitsbeaufschlagung ist bei Tampons mit Polypropylen deutlich geringer.

Insgesamt gesehen sind die Tampons aus 20 % Polypropylen und 8o % Zellwolle mit der erfindungsgemäßen Oberflächenvliesbildung aus folgenden Gründen besser zu bewerten als Tampons aus 100 % Zellwolle:

a) kein Abflusen

b) höhere Saugfähigkeit

c) besserer Seitenabschluß in der Vagina

d) reduzierte oder keine Druckbildung unter Tragebedingungen

Beispiel II:   Slipeinlagen

Das Herstellen von Slipeinlagen erfolgt an einer üblichen Slipeinlagenmaschine, wie sie zur Herstellung der
Einlagen auf Wattebasis üblich ist. Ein wie in Beispiel I
verfestigtes Watteband von ca. 130 $g/m^2$ wird in Wattestreifen 21 nach Fig. 2 von 100 mm Breite geschnitten.
Der Wattestreifen 21 besitzt eine erfindungsgemäß als
flusendichtes Vlies ausgebildete Oberfläche 22 und eine
offene Oberfläche 23. Im Verfahrensverlauf wird der Wattestreifen 21 in der angedeuteten Weise hälftig eingeschlagen, so daß die verfestigte Wattestreifenoberfläche
22 nach Fig. 3 die Außenseite bildet. Die Faltstelle bzw.
Faltlinie 24 nach Fig. 3 wird nach Fig. 4 mit einer
feuchtigkeitsundurchlässigen Folie 25 beklebt. Die Folie
25 dient als Wäscheschutzschicht. Auf die Folie 25 wird
wie üblich zum Befestigen an der Wäsche ein Haftstreifen
26, insbesondere mit einem silikonisierten Papier, aufgebracht. Mit Hilfe einer beheizten Stanzprägewalze können schließlich die Längsenden, insbesondere abgerundet,
gebildet und verfestigt werden, so daß sich flusendichte
Enden 27 ergeben. Das Stanzprägen und Verfestigen ist
in diesem Bereich möglich, da das Watteband thermoplastische Fasern bzw. Schmelzfasern enthält.

Das erfindungsgemäße Verfahren ist vor allem deshalb besonders wirtschaftlich, weil eine gesonderte Umhüllung
aus Vliesmaterial nicht erforderlich ist und damit zusammenhängende Störungen entfallen.

Patentanmeldung

HENKEL KGaA
ZR-FE/Patente

D 6837                        12

P a t e n t a n s p r ü c h e

1. Hygienevlies bestehend aus einem mit einem flusendichten Vliesstoff bedeckten Watteband (9, 21) gekennzeichnet durch eine heißgeprägte Oberflächenverfestigung (22) eines thermoplastischen Faseranteils des Wattebandes (21) als Vliesstoffdeckschicht.

2. Verfahren zum Herstellen eines Hygienevlieses bestehend aus einem mit einem flusendichten Vliesstoff bedeckten Watteband (9, 21), insbesondere nach Anspruch 1, dadurch gekennzeichnet, daß ein Watteband (9, 21) mit einem zum thermoplastischen Verfestigen der Oberfläche ausreichenden Kunststoffaseranteil vorbereitet wird (1 bis 8) und daß wenigstens eine Fläche des vorbereiteten Wattebandes (9, 21) in einem Kalander (10, 11) mit wenigstens einer eine beheizte Prägestruktur aufweisenden Arbeitswalze (17) vliesartig verfestigt wird.

3. Verfahren nach Anspruch 2, dadurch gekennzeichnet, daß der Anteil thermoplastisch zu verformender Fasern auf etwa 20 % der gesamten Fasermasse des Wattebandes (9, 21) eingestellt wird.

4. Verfahren nach Anspruch 2 oder 3, dadurch gekennzeichnet, daß die Temperatur der beheizten Arbeitswalze (10) abhängig von der Schmelztemperatur der zum Verfestigen heranzuziehenden thermoplastischen Faser gewählt wird.

5. Verfahren nach einem oder mehreren der Ansprüche 2 bis 4, dadurch gekennzeichnet, daß der Kalanderdruck und die Durchlaufgeschwindigkeit in etwa umgekehrt proportional gewählt werden.

...

0124834

D 6837                    13

6. Verfahren nach einem oder mehreren der Ansprüche 2 bis 5, dadurch gekennzeichnet, daß beide Flächen des Wattebandes (9) unmittelbar nacheinander auf zwei aufeinanderfolgenden Kalandern (10, 11) mit je einer ein Prägemuster aufweisenden Arbeitsweise (10) und einer Gegenwalze (11) vliesartig flusendicht verfestigt werden.

7. Verfahren nach einem oder mehreren der Ansprüche 2 bis 6, dadurch gekennzeichnet, daß die Wattebandlängskanten und/oder die Trennlinien der im Watteband aufeinanderfolgenden Abschnitte im Thermoschneidverfahren bearbeitet und flusendicht verfestigt werden.

8. Verfahren nach einem oder mehreren der Ansprüche 2 bis 7, dadurch gekennzeichnet, daß die Wattebandoberfläche mit einem Linien und/oder Punkte enthaltenden Prägemuster heißgeprägt wird.

D 6837

## Fig.1

1
2
4
3
2
5
2
6
7
8
9
2
10
11
11
10
12

## Fig.2

21
22
23

## Fig.3

22
23
24
22

## Fig.4

25

## Fig.5

26

## Fig.6

27
22
27

HENKEL KGaA
ZR-FE/Patente
0124834